# EUROPEAN PATENT APPLICATION

(11) **EP 3 603 416 A1**
(43) Date of publication of application: **05.02.2020**
(21) Application number: 18774700.1
(22) Date of filing: 22.03.2018
(51) Int. Cl.: A23L 21/10

(54) **JELLY CONTAINING PYRROLOQUINOLINE QUINONE**

(30) Priority: 28.03.2017 JP 2017062594
(71) Applicant: Mitsubishi Gas Chemical Company, Inc., Tokyo 100-8324 (JP)
(72) Inventor: TAMAKOSHI, Masanori, Niigata-shi Niigata 950-3112 (JP); SHINBORI, Takeshi, Niigata-shi Niigata 950-3112 (JP); IMARUOKA, Satoko, Niigata-shi Niigata 950-3112 (JP); IKEMOTO, Kazuto, Niigata-shi Niigata 950-3112 (JP)
(74) Representative: Müller-Boré & Partner Patentanwälte PartG mbB
(86) International application number: PCT/JP2018/011390
(87) International publication number: WO 2018/180885

(57) **Abstract**

The present invention provides a jelly containing pyrroloquinoline quinone and/or a salt thereof, a non-reducing sugar and a gelling agent.

## Description

### Technical Field

The present invention relates to a pyrroloquinoline quinone-containing jelly, a method for producing the same, and the like.

### Background Art

Pyrroloquinoline quinone (hereinafter sometimes referred to as "PQQ") has been known as a microbial coenzyme, and its known functions include brain function improvement, mitochondrial activation and cell proliferation.

Pyrroloquinoline quinone can be blended as a watersoluble disodium salt when added to food. For example, in Patent Literature 1, carbonated water blended with a pyrroloquinoline quinone disodium salt is specifically described.

### Citation List

### Patent Literature

Patent Literature 1: Japanese Patent Laid-Open No. 2013-237644
Patent Literature 2: International Publication No. WO 2012/020767
Patent Literature 3: Japanese Patent Laid-Open No. 2013-103913

### Summary of Invention

### Technical Problem

A salt of PQQ is known to form a fibrous structure and eventually form a gel by mixing its aqueous solution with a reducing agent at room temperature (Patent Literature 2). However, there is no known example in which PQQ is blended in a jelly such as gel. To provide a jelly containing PQQ, it is necessary to examine its stability, etc. as a gel food, but there are many types of gelling agents, and there is no information regarding gelation in the presence of pyrroloquinoline quinone.

When provided as a dessert, gels often have a sweet taste. Therefore, in order to include PQQ in a gel, it is necessary to examine the stability of the gel in the presence of a sweetener besides the gelling agent. Typical substances for sweeteners are sugars, but their interaction with pyrroloquinoline quinone is unknown. Moreover, there is no information regarding the effect of sweeteners on the gel stability, either.

An object of the present invention is to provide a stable jelly blended with pyrroloquinoline quinone.

### Solution to Problem

Usually, the type of sugar does not affect the gelation. However, as a result of intensive studies to achieve the above object, the present inventors have found that, surprisingly, gelation does not occur depending on the type of sugar when PQQ is added, and even when gelation occurs, the stability of the jelly also changes depending on the type of sugar. Furthermore, PQQ easily colors the tongue and the skin, and a method of using PQQ in combination with a chelating agent has been proposed to prevent coloring (Patent Literature 3), but it became clear that coloring can be suppressed without any chelating agent by using a specific sugar. That is, the inventors have found that using a specific sugar is unexpectedly important for producing a pyrroloquinoline quinone-containing jelly, and has completed the present invention.

That is, the present invention is as follows.
(1) A jelly comprising:
   pyrroloquinoline quinone and/or a salt thereof;
   a non-reducing sugar; and
   a gelling agent.
(2) The jelly according to (1), comprising:
   0.004 to 0.05 percent by weight of pyrroloquinoline quinone and/or a salt thereof;
   2 to 20 percent by weight of the non-reducing sugar; and
   0.2 to 5 percent by weight of the gelling agent.
(3) The jelly according to (1), wherein a salt of pyrroloquinoline quinone is a pyrroloquinoline quinone disodium salt.
(4) The jelly according to any one of (1) to (3), wherein the non-reducing sugar is sorbitol, xylitol or sucrose.
(5) The jelly according to any one of (1) to (4), wherein the gelling agent is agar or gelatin.
(6) The jelly according to any one of (1) to (5), wherein the jelly is homogenized.
(7) A method for producing the jelly according to any one of (1) to (6), comprising a step of blending pyrroloquinoline quinone and/or a salt thereof, a non-reducing sugar and a gelling agent.
(8) A stabilizer for the jelly containing pyrroloquinoline quinone or a salt thereof, comprising a non-reducing sugar as an active ingredient.
(9) A coloring inhibitor for the jelly containing pyrroloquinoline quinone or a salt thereof, comprising a non-reducing sugar as an active ingredient.

### Advantageous Effects of Invention

According to the present invention, it becomes possible to stabilize pyrroloquinoline quinone-containing jelly and to suppress the coloring specific to pyrroloquinoline quinone by blending with a non-reducing sugar. As a result, functional foods containing pyrroloquinoline quinone can be ingested in the form of an easy-to-eat jelly.

As sweeteners to be blended with gels that are often eaten at low temperatures such as desserts, isomerized sugars that hardly change with temperature are often used. Unexpectedly, the investigation by the present inventors showed for the first time that, when an isomerized sugar, which is a reducing sugar, is blended, the stability of the jelly containing pyrroloquinoline quinone is reduced, whereas the stability improves when a non-reducing sugar is used.

### Description of Embodiments

Hereinafter, the embodiments of the present invention (hereinafter, simply referred to as "the present embodiments") will be described in detail, but the present invention is not limited to the following present embodiments. The present invention can be variously modified without deviating from the scope of the invention.

The pyrroloquinoline quinone-containing jelly of the present invention contains pyrroloquinoline quinone and/or a salt thereof and a non-reducing sugar. The jelly of the present invention is gelled by a gelling agent. That is, as used herein, "jelly" means a jelly such as a gel formed by the gelling ability possessed by commonly used gelling agents such as agar or gelatin, and gels formed by the gel-forming ability of pyrroloquinoline quinone itself using a dispersion medium, as described in International Publication No. WO 2012/020767 (supra), are not intended to be included within the scope of the present invention.

The pyrroloquinoline quinone used in the present invention is a substance having the structure shown in Formula 1. In the present invention, not only a free form of pyrroloquinoline quinone but also a salt thereof can be used.

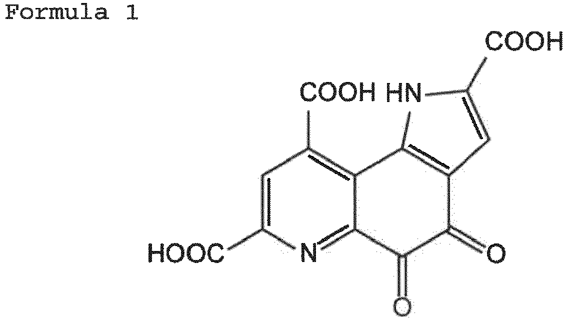

The pyrroloquinoline quinone or a salt thereof used in the present invention can be obtained commercially, but it can also be produced by a known method. Examples of a salt of pyrroloquinoline quinone include alkali metal salts, alkaline earth metal salts and ammonium salts of pyrroloquinoline quinone. A preferable salt is an alkali metal salt.

Examples of the alkali metal salt of pyrroloquinoline quinone used in the present invention include salts of sodium, potassium, lithium, cesium and rubidium. Preferably, a sodium salt or a potassium salt is more preferable in terms of easy availability. The alkali metal salt of pyrroloquinoline quinone may be an alkali metal salt substituted with 1 to 3 alkali metal atoms, and may be any one of a mono-alkali metal salt, a di-alkali metal salt or a tri-alkali metal salt. Preferably, a di-alkali metal salt is used. Particularly preferably, a disodium salt and a dipotassium salt are used. Among these, hydrated crystals of a disodium salt are preferable since they have a high stability and are easy to use.

Various gelling agents can be used as long as they can form a jelly containing pyrroloquinoline quinone. However, when 0.2 to 5 percent by weight, for example, 2 percent by weight of the gelling agent is added based on the total weight of the jelly, agar or gelatin is preferable because it easily forms a jelly. The amount of agar or gelatin is more preferably about 0.4 to 2 percent by weight. It is preferable that the active ingredients such as agar or gelatin be contained in an amount of 80 percent by weight or more in the gelling agent. Agar or gelatin is further preferable since it promotes gelation without coloring even in the presence of pyrroloquinoline quinone. The gelling agent is not limited to agar and gelatin, and other gelling agents may be used alone or in combination with agar or gelatin, as long as they can form a gel.

The gelling agent used in the present invention can be used as long as it can form a gel, regardless of the origin and treatment method of the raw materials.

The non-reducing sugar blended as a sweetener suppresses the color change of pyrroloquinoline quinone and contributes to the stabilization of the gel. The non-reducing sugar is a sugar that does not form a ketone or an aldehyde under basic conditions. Specific example thereof includes sorbitol, xylitol, palatinit, sucrose and reducing starch syrup. In general, the gelation progresses when using a reducing sugar such as glucose, but the use of a reducing sugar in the present invention brought about an unexpected effect of making gelation difficult. However, the addition of a reducing sugar is not to be completely excluded, and it is acceptable to add a reducing sugar in an amount that does not inhibit the desired effect.

In one embodiment, the jelly of the present invention contains 0.004 to 0.05 percent by weight of pyrroloquinoline quinone and/or a salt thereof. (the description of "... to ..." includes both upper limit value and lower limit value, and the same applies hereinafter). By setting the amount of pyrroloquinoline quinone blended in this range, coloring on the tongue can be suppressed. In a preferable embodiment, the amount of pyrroloquinoline quinone and/or a salt thereof blended in the jelly may be 0.01 to 0.04 percent by weight. It is preferable to ingest pyrroloquinoline quinone and/or a salt thereof in an amount of 10 to 200 mg in terms of pyrroloquinoline quinone disodium trihydrate per day for an adult. In particular, from the viewpoint of improving brain function, the amount of the pyrroloquinoline quinone and/or a salt thereof blended or the amount of jelly may be adjusted so that the pyrroloquinoline quinone and/or a salt thereof be 2 to 200 mg, preferably 5 to 50 mg, in terms of pyrroloquinoline quinone disodium trihydrate per day for an adult. The jelly of the present invention may contain 2 to 20 percent by weight of a non-reducing sugar sweetener in terms of sucrose based on the total weight. By setting it to this range, moderate sweetness can be obtained so that good taste is felt.

Homogenizing the jelly of the present invention could produce jelly sweets with fluidity. A fluid jelly has an excellent texture and has the advantage of being able to be ingested in a short time.

The following components can be optionally added to the pyrroloquinoline quinone-containing jelly of the present invention, as needed, to such an extent that the effects of the present invention and the flavor of the jelly are not adversely affected.

There are no particular limitations on the use of acidulants, flavoring agents, coloring agents and the like.

Functional materials such as dietary fiber, vitamins, minerals and amino acids, fats and oils, emulsifiers, dairy products, high-sweetness sweeteners (aspartame, neotame, glycyrrhizin, saccharin, stevioside, rebaudio, dulcin, alitame, trichlorosucrose, thaumatin, acesulfame potassium, sucralose and the like) and the like are used.

In the case of producing the jelly of the present invention, the gelling agent and the non-reducing sugar are first dissolved in water or in a buffer solution, and the resulting solution is heated to prepare a gelling agent solution. Next, by mixing the gelling agent solution with an aqueous pyrroloquinoline quinone solution, the jelly of the present invention can be produced. As a buffer solution that can be used for the preparation of the gelling agent solution, any aqueous solution prepared from a pH adjuster that is allowed to be added to food may be used, for example, phosphate buffer and citrate buffer can be used. The aqueous solution of pyrroloquinoline quinone having a concentration of 0.004 percent by weight to 0.2 percent by weight can be used.

In the case of using gelatin as the gelling agent, it is preferable to mix at 30 to 50°C in order to increase the stability of pyrroloquinoline quinone. The temperature at which gelatin is mixed may be arbitrary, but it is preferable to be 50 to 120°C in consideration of the temperature at which gelatin dissolves. The pyrroloquinoline quinone and/or a salt thereof, the gelling agent and the non-reducing sugar may be mixed simultaneously. When mixed simultaneously, it is preferable not to leave it at a high temperature for a long time in order to improve the stability of PQQ.

It is preferable that the amount of the jelly of the present invention to be ingested be 10 to 50 mg in terms of pyrroloquinoline quinone disodium trihydrate per day. With this amount of ingestion, it becomes a functional food that can be expected to moisturize the skin, improve brain function and improve the lipids.

In another aspect, the present invention provides a stabilizer for the jelly containing pyrroloquinoline quinone or a salt thereof, comprising a non-reducing sugar as an active ingredient. The content of the active ingredient is the same as the amount blended in the above jelly.

Furthermore, in another aspect, the present invention provides a coloring inhibitor for the jelly containing pyrroloquinoline quinone or a salt thereof, comprising a non-reducing sugar as an active ingredient.

### Examples

Hereafter, the present invention is further described in detail with Examples, but the present invention is not limited to these Examples.

As pyrroloquinoline quinone disodium, BioPQQ (registered trademark) manufactured by Mitsubishi Gas Chemical Company, Inc. was used. This product is crystalline and the trihydrate is stable. The other reagents used were manufactured by Wako Pure Chemical Industries, Ltd.

### Example 1

A sample (total weight: 50 g) with a composition consisting of 0.02 percent by weight of pyrroloquinoline quinone disodium, 10 percent by weight of sorbitol, 2 percent by weight of agar and the balance being water was prepared and heated in an autoclave (121°C) for 15 minutes. The heated solution was homogenized by mixing. The resulting solution was cooled in a refrigerator (4°C) and stored for one day to obtain a jelly food. The resulting jelly had a red color due to the pyrroloquinoline quinone, and the color was uniform as a whole.

Analysis of the pyrroloquinoline quinone in the jelly
1 g of each jelly was mixed with 10 mL of water, placed in a 15 mL polypropylene centrifugation container, and shaken at room temperature for 30 minutes. This solution was diluted 10 times with an eluent for liquid chromatography. The resultant was filtered through a 0.2 pm filter. The pyrroloquinoline quinone content was analyzed using high performance liquid chromatography (HPLC) under the following conditions.
HPLC conditions: 259 nm, 40°C, YMC-Pack ODS-A 150 mm, 4.6 mm, 30 mM acetic acid - 70 mM ammonium acetate, apparatus LC2010 (manufactured by Shimadzu Corporation)

As a result, the content of pyrroloquinoline quinone disodium added was retained at 100% in the jelly.

### Comparative Example 1

As isomerized sugar, isomerized sugar HC (55% or more fructose content) manufactured by Oji Cornstarch Co., Ltd. was used. A sample (total weight: 50 g) with a composition consisting of 0.02 percent by weight of pyrroloquinoline quinone disodium, 10 percent by weight of isomerized sugar, 2 percent by weight of agar and the balance water was prepared, heated in an autoclave (121°C) for 15 minutes, and homogenized. The resulting solution was cooled in a refrigerator (4°C). Even after one day, the solution was not solidified in a jelly-like state and the color of the mixture that was initially red had turned yellow. In addition, according to the HPLC analysis, the pyrroloquinoline quinone disodium content in the jelly had been reduced to 59% relative to the addition amount.

### Examples 2 to 5, Comparative Examples 2 to 4

A jelly food in which only the content of pyrroloquinoline quinone disodium was changed in the composition of the sample of Example 1 was prepared according to the same method as in Example 1 (Examples 2 to 5). Similarly, a jelly food in which only the content of pyrroloquinoline quinone disodium was changed in the composition of the sample of Comparative Example 1 was prepared according to the same method as in Comparative Example 1 (Comparative Example 2). In addition, jelly foods to which glucose (Comparative Example 3) or fructose (Comparative Example 4) was added instead of the isomerized sugar of Comparative Example 1 were also prepared. 5 g of each prepared jelly food was put in the mouth and held for 30 seconds, then the ease of coloring of pyrroloquinoline quinone on the tongue was observed by looking at the whole tongue 5 minutes later.

The gelation was evaluated by visual observation on a three-level scale; A: solidified, B: solidified but soft, C: solidified but very soft and solid substances and solution are mixed. The results are shown in Table 1.

**[Table 1]**

| | PQQ disodium (percent by weight) | Sweetener (10 percent by weight) | Gelling agent (2 percent by weight) | Gelation evaluation | PQQ content in gel (%) | Coloring on tongue |
|---|---|---|---|---|---|---|
| Example 1 | 0.02 | Sorbitol | Agar | A | 100 | No |
| Example 2 | 0.004 | Sorbitol | Agar | A | 100 | No |
| Example 3 | 0.01 | Sorbitol | Agar | A | 100 | No |
| Example 4 | 0.02 | Sucrose | Agar | A | 100 | No |
| Example 5 | 0.02 | Xylitol | Agar | A | 100 | No |
| Comparative Example 2 | 0.004 | Isomerized sugar | Agar | C | 25 | No* |
| Comparative Example 3 | 0.02 | Glucose | Agar | B | 71 | No* |
| Comparative Example 4 | 0.02 | Fructose | Agar | C | 67 | No* |

| | | | | | | |
|---|---|---|---|---|---|---|
| *) The color of jelly changed from red to yellow over time | | | | | | |

When sucrose, xylitol or sorbitol as a non-reducing sugar was added, a stable jelly containing pyrroloquinoline quinone could be made. On the other hand, unexpectedly, adding isomerized sugar, glucose or fructose as a reducing sugars resulted in a low stability of the jelly. In particular, when the amount of pyrroloquinoline quinone blended was small, the pyrroloquinoline quinone content in the gel decreased. No coloring on the tongue was observed for any of the jellies, but in the presence of a reducing sugar, the color of the jelly also changed from red to yellow over time. Despite a very high agar concentration, the jerry hardly turned into a gel when using reducing sugars, and in particular, no gel was formed with isomerized sugars and fructose. Although gelation was observed when glucose was added, the formed jelly was soft.

### Examination of the type of gelling agent Examples 6 and 7, Reference Examples 1 to 3

The formation of jelly was examined by changing the type of the gelling agent. A sample (total weight: 50 g) with a composition consisting of 0.02 percent by weight of pyrroloquinoline quinone disodium, 10 percent by weight of sorbitol, 2 percent by weight of gelling agent and the balance being water was prepared, heated in an autoclave (121°C) for 15 minutes and homogenized. The resultant was cooled in a refrigerator (4°C) and the formation of jelly was observed one day later. The results are shown in Table 2.

**[Table 2]**

| | Gelling agent | Appearance after one day |
|---|---|---|
| Example 6 | Agar | Jelly formed |
| Example 7 | Gelatin | Jelly formed |
| Reference Example 1 | Carrageenan | Not solidified, color changed to orange |
| Reference Example 2 | Gum Arabic | Not solidified, color changed to orange |
| Reference Example 3 | Xanthan gum | Jelly with color changed to green and inhomogeneously solidified |

Besides agar, gelatin was suitable for the jelly. The content of pyrroloquinoline quinone in the jelly of Example 7, which was gelled with gelatin, was 86%. The results are not shown, but it was verified that, when the conditions used in the above Reference Examples were changed, for example, when the amount of carrageenan, gum arabic or xanthan gum, or the like was increased, all turned into a gel.

### Comparative Example 5

Based on Example 13 of International Publication No. WO 2012/020767 (supra), a gel of pyrroloquinoline quinone itself was prepared using a salt of pyrroloquinoline quinone and a dispersion medium. First, 0.1 g of PQQ powder was added to a 15 mL plastic centrifugation container, 10 ml of water was added thereto, and the mixture was mixed by shaking at room temperature (about 25°C). The resultant was cooled in a refrigerator to 4°C, then further shaken to mix. When the resultant was stored in a refrigerator overnight, it turned entirely into a uniform gel. Next, 0.01 g of agar was added to 10 mL of water and dissolved in a microwave oven, the resultant was cooled to about 40°C, and 1 mL each was mixed with the above pyrroloquinoline quinone 1% gel. When the mixture was cooled overnight in a refrigerator, it gelled, and fibers were observed by an optical microscope. The resulting jelly was put in the mouth and held for 30 seconds, and the color of the jelly colored the tongue.

### Example 8 Crushed jelly

Using a homogenizer, the jelly made in Example 1 was crushed so as to break into a uniform size at room temperature. The solidified jelly was crushed into a fluid crushed jelly.

### Example 9-1, Example 9-2 and Comparative Example 6

The concentration of pyrroloquinoline quinone disodium was varied to examine coloring on the tongue. Preparation of the jelly was performed as described above, except that the amounts were fixed at 10 percent by weight of sorbitol and 2% of agar.

**[Table 3]**

| | PQQ disodium (percent by weight) | Coloring |
|---|---|---|
| Example 9-1 | 0.02 | No |
| Example 9-2 | 0.04 | No |
| Comparative Example 6 | 0.06 | Yes |

It became clear that when the amounts of sorbitol and agar are 10 percent by weight and 2%, respectively, the amount of PQQ blended of about 0.02 to 0.04 percent by weight did not cause coloring on the tongue.

### Comparative Example 7 Example in aqueous solution

As a result of examining coloring by putting an aqueous solution of 0.04 percent by weight of pyrroloquinoline quinone disodium and 10 percent by weight of sorbitol in the mouth for 30 seconds, coloring was observed on the tongue. From these results, it has been surprisingly found that non-reducing sugars need to be blended with the jelly to suppress the coloring of pyrroloquinoline quinone.

### Industrial Applicability

The jelly of the present invention is useful for supplements, functional foods, jelly complex sweets and cosmetics.

## Claims

1. A jelly comprising:
pyrroloquinoline quinone and/or a salt thereof;
a non-reducing sugar; and
a gelling agent.

2. The jelly according to claim 1, comprising:
0.004 to 0.05 percent by weight of pyrroloquinoline quinone and/or a salt thereof;
2 to 20 percent by weight of the non-reducing sugar; and
0.2 to 5 percent by weight of the gelling agent.

3. The jelly according to claim 1 or 2, wherein a salt of pyrroloquinoline quinone is a pyrroloquinoline quinone disodium salt.

4. The jelly according to any one of claims 1 to 3, wherein the non-reducing sugar is sorbitol, xylitol or sucrose.

5. The jelly according to any one of claims 1 to 4, wherein the gelling agent is agar or gelatin.

6. The jelly according to any one of claims 1 to 5, wherein the jelly is homogenized.

7. A method for producing the jelly according to any one of claims 1 to 6, comprising a step of blending pyrroloquinoline quinone and/or a salt thereof, a non-reducing sugar and a gelling agent.

8. A stabilizer for the jelly containing pyrroloquinoline quinone or a salt thereof, comprising a non-reducing sugar as an active ingredient.

9. A coloring inhibitor for the jelly containing pyrroloquinoline quinone or a salt thereof, comprising a non-reducing sugar as an active ingredient.
